# EUROPEAN PATENT SPECIFICATION

(11) **EP 0 870 827 B1**
(45) Date of publication and mention of the grant of the patent: **03.03.2004**
(21) Application number: 97310562.0
(22) Date of filing: 23.12.1997
(51) Int. Cl.: C12N 15/12, C07K 14/715, C07K 16/28, A61K 38/17, C12Q 1/68, G01N 33/68

(54) **Tumor necrosis factor related receptor, TR6**
Tumor-Nekrosis-Faktor verwandter Receptor, TR6
Récepteur apparenté au facteur de necrose tumorale, TR6

(30) Priority: 14.03.1997 US 41230 P; 09.05.1997 US 853684; 22.08.1997 US 916625
(43) Date of publication of application: 14.10.1998
(73) Proprietor: SMITHKLINE BEECHAM CORPORATION, Philadelphia Pennsylvania 19103 (US)
(72) Inventor: Deen, Keith Charles, King of Prussia, Pennsylvania 19406 (US); Young, Peter Ronald, King of Prussia, Pennsylvania 19406 (US)
(74) Representative: Crump, Julian Richard John

(56) References cited:
- EP-A- 0 510 691
- WO-A-97/01633
- WO-A-98/46643
- SHERIDAN J P ET AL: "Control of TRAIL-induced apoptosis by a family of signaling and decoy receptors" SCIENCE, vol. 277, 8 August 1997, pages 818-821, XP002075799
- PAN G ET AL: "AN ANTAGONIST DECOY RECEPTOR AND A DEATH DOMAIN-CONTAINING RECEPTOR FOR TRAIL" SCIENCE, vol. 277, 8 August 1997, pages 815-818, XP002065147
- CHINNAIYAN A M ET AL: "SIGNAL TRANSDUCTION BY DR3, A DEATH DOMAIN-CONTAINING RECEPTOR RELATED TO TNFR-1 AND CD95" SCIENCE, vol. 274, no. 5289, 8 November 1996, pages 990-992, XP000676685
- BUCHER P. ET AL: 'A flexible motif search technique based on generalized profiles' COMPUTERS CHEMISTRY vol. 20, no. 1, 1996, pages 3 - 23
- DATABASE EMBL [Online] EBI 19 February 1997 HILLIER ET AL.: 'EST' Database accession no. (AA223122)

### SEQUENCE LISTING

### (1) GENERAL INFORMATION

(i) APPLICANT: SmithKline Beecham Corporation
(ii) TITLE OF THE INVENTION: TUMOR NECROSIS FACTOR RELATED RECEPTOR, TR6
(iii) NUMBER OF SEQUENCES: 6
(iv) CORRESPONDENCE ADDRESS:
   (A) ADDRESSEE: SmithKline Beecham,
      Corporate Intellectual Property
   (B) STREET: Two New Horizons Court
   (C) CITY: Brentford
   (D) COUNTY: Middles ex
   (E) COUNTRY: United Kingdom
   (F) POST CODE: TW8 9EP
(v) COMPUTER READABLE FORM:
   (A) MEDIUM TYPE: Diskette
   (B) COMPUTER: IBM Compatible
   (C) OPERATING SYSTEM: DOS
   (D) SOFTWARE: FastSEQ for Windows Version 2.0
(vi) CURRENT APPLICATION DATA:
   (A) APPLICATION NUMBER TO BE ASSIGNED
   (B) FILING DATE: 22-AUGUST-1997
   (C) CLASSIFICATION: Unknown
(vii) PRIOR APPLICATION DATA:
   (A) APPLICATION NUMBER 08/853,684
   (B) FILING DATE: 09-MAY-1997
(viii) ATTORNEY/AGENT INFORMATION:
   (A) NAME: THOMPSON, Clive Beresford
   (B) GENERAL AUTHORISATION NUMBER 5630
   (C) REFERENCE/DOCKET NUMBER: GH-50008-1
(ix) TELECOMMUNICATION INFORMATION:
   (A) TELEPHONE: +44 181 975 6347
   (B) TELEFAX: +44 181 975 6294
   (C) TELEX:

### (2) INFORMATION FOR SEQ ID NO: 1:

(i) SEQUENCE CHARACTERISTICS:
   (A) LENGTH: 3,881 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO: 1:
   CTTTGCGCCC ACAAAATACA CCGACGATGC CCGATCTACT TTAAGGGCTG AAACCCACGG    60
   GCCTGAGAGA CTATAAGAGC GTTCCCTACC GCCATGGAAC AACGGGGACA GAACGCCCCG   120
   GCCGCTTCGG GGGCCCGGAA AAGGCACGGC CCAGGACCCA GGGAGGCGCG GGGAGCCAGG   180
   CCTGGGCCCC GGGTCCCCAA GACCCTTGTG CTCGTTGTCG CCGCGGTCCT GCTGTTGGTC   240
   TCAGCTGAGT CTGCTCTGAT CACCCAACAA GACCTAGCTC CCCAGCAGAG AGCGGCCCCA   300
   CAACAAAAGA GGTCCAGCCC CTCAGAGGGA TTGTGTCCAC CTGGACACCA TATCTCAGAA   360
   GACGGTAGAG ATTGCATCTC CTGCAAATAT GGACAGGACT ATAGCACTCA ATGGAATGAC   420
   CTCCTTTTCT GCTTGCGCTG CACCAGGTGT GATTCAGGTG AAGTGGAGCT AAGTCCCTGC   480
   ACCACGACCA GAAACACAGT GTGTCAGTGC GAAGAAGGCA CCTTCCGGGA AGAAGATTCT   540
   CCTGAGATGT GCCGGAAGTG CCGCACAGGG TGTCCCAGAG GGATGGTCAA GGTCGGTGAT   600
   TGTACACCCT GGAGTGACAT CGAATGTGTC CACAAAGAAT CAGGCATCAT CATAGGAGTC   660
   ACAGTTGCAG CCGTAGTCTT GATTGTGGCT GTGTTTGTTT GCAAGTCTTT ACTGTGGAAG   720
   AAAGTCCTTC CTTACCTGAA AGGCATCTGC TCAGGTGGTG GTGGGGACCC TGAGCGTGTG   780
   GACAGAAGCT CACAACGACC TGGGGCTGAG GACAATGTCC TCAATGAGAT CGTGAGTATC 840
   TTGCAGCCCA CCCAGGTCCC TGAGCAGGAA ATGGAAGTCC AGGAGCCAGC AGAGCCAACA 900
   GGTGTCAACA TGTTGTCCCC CGGGGAGTCA GAGCATCTGC TGGAACCGGC AGAAGCTGAA 960
   AGGTCTCAGA GGAGGAGGCT GCTGGTTCCA GCAAATGAAG GTGATCCCAC TGAGACTCTG 1020
   AGACAGTGCT TCGATGACTT TGCAGACTTG GTGCCCTTTG ACTCCTGGGA GCCGCTCATG 1080
   AGGAAGTTGG GCCTCATGGA CAATGAGATA AAGGTGGCTA AAGCTGAGGC AGCGGGCCAC 1140
   AGGGACACCT TGTACACGAT GCTGATAAAG TGGGTCAACA AAACCGGGCG AGATGCCTCT 1200
   GTCCACACCC TGCTGGATGC CTTGGAGACG CTGGGAGAGA GACTTGCCAA GCAGAAGATT 1260
   GAGGACCACT TGTTGAGCTC TGGAAAGTTC ATGTATCTAG AAGGTAATGC AGACTCTGCC 1320
   ATGTCCTAAG TGTGATTCTC TTCAGGAAGT CAGACCTTCC CTGGTTTACC TTTTTTCTGG 1380
   AAAAAGCCCA ACTGGACTCC AGTCAGTAGG AAAGTGCCAC AATTGTCACA TGACCGGTAC 1440
   TGGAAGAAAC TCTCCCATCC AACATCACCC AGTGGATGGA ACATCCTGTA ACTTTTCACT 1500
   GCACTTGGCA TTATTTTTAT AAGCTGAATG TGATAATAAG GACACTATGG AAATGTCTGG 1560
   ATCATTCCGT TTGTGCGTAC TTTGAGATTT GGTTTGGGAT GTCATTGTTT TCACAGCACT 1620
   TTTTTATCCT AATGTAAATG CTTTATTTAT TTATTTGGGC TACATTGTAA GATCCATCTA 1680
   CACAGTCGTT GTCCGACTTC ACTTGATACT ATATGATATG AACCTTTTTT GGGTGGGGGG 1740
   TGCGGGGCAG TTCACTCTGT CTCCCAGGCT GGAGTGCAAT GGTGCAATCT TGGCTCACTA 1800
   TAGCCTTGAC CTCTCAGGCT CAAGCGATTC TCCCACCTCA GCCATCCAAA TAGCTGGGAC 1860
   CACAGGTGTG CACCACCACG CCCGGCTAAT TTTTTGTATT TTGTCTAGAT ATAGGGGCTC 1920
   TCTATGTTGC TCAGGGTGGT CTCGAATTCC TGGACTCAAG CAGTCTGCCC ACCTCAGACT 1980
   CCCAAAGCGG TGGAATTAGA GGCGTGAGCC CCCATGCTTG GCCTTACCTT TCTACTTTTA 2040
   TAATTCTGTA TGTTATTATT TTATGAACAT GAAGAAACTT TAGTAAATGT ACTTGTTTAC 2100
   ATAGTTATGT GAATAGATTA GATAAACATA AAAGGAGGAG ACATACAATG GGGGAAGAAG 2160
   AAGAAGTCCC CTGTAAGATG TCACTGTCTG GGTTCCAGCC CTCCCTCAGA TGTACTTTGG 2220
   CTTCAATGAT TGGCAACTTC TACAGGGGCC AGTCTTTTGA ACTGGACAAC CTTACAAGTA 2280
   TATGAGTATT ATTTATAGGT AGTTGTTTAC ATATGAGTCG GGACCAAAGA GAACTGGATC 2340
   CACGTGAAGT CCTGTGTGTG GCTGGTCCCT ACCTGGGCAG TCTCATTTGC ACCCATAGCC 2400
   CCCATCTATG GACAGGCTGG GACAGAGGCA GATGGGTTAG ATCACACATA ACAATAGGGT 2460
   CTATGTCATA TCCCAAGTGA ACTTGAGCCC TGTTTGGGCT CAGGAGATAG AAGACAAAAT 2520
   CTGTCTCCCC ACGTCTGCCA TGGCATCAAG GGGGAAGAGT AGATGGTGCT TGAGAATGGT 2580
   GTGAAATGGT TGCCATCTCA GGAGTAGATG GCCCGGCTCA CTTCTGGTTA TCTGTCACCC 2640
   TGAGCCCATG AGCTGCCTTT TAGGGTACAG ATTGCCTACT TGAGGACCTT GGCCGCTCTG 2700
   TAAGCATCTG ACTCATCTCA GAAATGTCAA TTCTTAAACA CTGTGGCAAC AGGACCTAGA 2760
   ATGGCTGACG CATTAAGGTT TTCTTCTTGT GTCCTGTTCT ATTATTGTTT TAAGACCTCA 2820
   GTAACCATTT CAGCCTCTTT CCAGCAAACC CTTCTCCATA GTATTTCAGT CATGGAAGGA 2880
   TCATTTATGC AGGTAGTCAT TCCAGGAGTT TTTGGTCTTT TCTGTCTCAA GGCATTGTGT 2940
   GTTTTGTTCC GGGACTGGTT TGGGTGGGAC AAAGTTAGAA TTGCCTGAAG ATCACACATT   3000
   CAGACTGTTG TGTCTGTGGA GTTTTAGGAG TGGGGGGTGA CCTTTCTGGT CTTTGCACTT   3060
   CCATCCTCTC CCACTTCCAT CTGGCATCCC CACGCGTTGT CCCCTGCACT TCTGGAAGGC   3120
   ACAGGGTGCT GCTGCTTCCT GGTCTTTGCC TTTGCTGGGC CTTCTGTGCA GGACGCTCAG   3180
   CCTCAGGGCT CAGAAGGTGC CAGTCCGGTC CCAGGTCCCT TGTCCCTTCC ACAGAGGCCT   3240
   TCCTAGAAGA TGCATCTAGA GTGTCAGCCT TATCAGTGTT TAAGATTTTT CTTTTATTTT   3300
   TAATTTTTTT GAGACAGAAT CTCACTCTCT CGCCCAGGCT GGAGTGCAAC GGTACGATCT   3360
   TGGCTCAGTG CAACCTCCGC CTCCTGGGTT CAAGCGATTC TCGTGCCTCA GCCTCCGGAG   3420
   TAGCTGGGAT TGCAGGCACC CGCCACCACG CCTGGCTAAT TTTTGTATTT TTAGTAGAGA   3480
   CGGGGTTTCA CCATGTTGGT CAGGCTGGTC TCGAACTCCT GACCTCAGGT GATCCACNTT   3540
   GGCCTCCGAA AGTGCTGGGA TATACAAGGC GTGAGCCACC AGCCAGGCCA AGATATTNTT   3600
   NTAAAGNNAG CTTCCGGANG ACATGAAATA ANGGGGGGTT TTGTTGTTTA GTAACATTNG   3660
   GCTTTGATAT ATCCCCAGGC CAAATNGCAN GNGACACAGG ACAGCCATAG TATAGTGTGT   3720
   CACTCGTGGT TGGTGTCCTT TCATGGTTCT GCCCTGTCAA AGGTCCCTAT TTGAAATGTG   3780
   TTATAATACA AACAAGGAAG CACATTGTGT ACAAAATACT TATGTATTTA TGAATCCATG   3840
   ACCAAATTAA ATATGAAACC TTATATAAAA AAAAAAAAAA A                       3881

### (2) INFORMATION FOR SEQ ID NO: 2:

(i) SEQUENCE CHARACTERISTICS:
   (A) LENGTH: 411 amino acids
   (B) TYPE: amino aci d
   (C) STRANDEDNESS: single
   (D) TOPOLOGY: linear
(ii) MOLECULE TYPE: protein
(xi) SEQUENCE DESCRIPTION: SEQ ID NO: 2:

### (2) INFORMATION FOR SEQ ID NO: 3:

(i) SEQUENCE CHARACTERISTICS:
   (A) LENGTH: 1062 base pai rs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO: 3:
   ATGACCTCCT TTTCTGCTTG CGCTGCACCA GGTGTGATTC AGGTGAAGTG GAGCTAAGTC    60
   CCTGCACCAC GACCAGAAAC ACAGTGTGTC AGTGCGAAGA AGGCACCTTC CGGGAAGAAG   120
   ATTCTCCTGA GATGTGCCGG AAGTGCCGCA CAGGGTGTCC CAGAGGGATG GTCAAGGTCG   180
   GTGATTGTAC ACCCTGGAGT GACATCGAAT GTGTCCACAA AGAATCAGGC ATCATCATAG   240
   GAGTCACAGT TGCAGCCGTA GTCTTGATTG TGGCTGTGTT TGTTTGCAAG TCTTTACTGT   300
   GGAAGAAAGT CCTTCCTTAC CTGAAAGGCA TCTGCTCAGG TGGTGGTGGG GACCCTGAGC   360
   GTGTGGACAG AAGCTCACAA CGACCTGGGG CTGAGGACAA TGTCCTCAAT GAGATCGTGA   420
   GTATCTTGCA GCCCACCCAG GTCCCTGAGC AGGAAATGGA AGTCCAGGAG CCAGCAGAGC   480
   CAACAGGTGT CAACATGTTG TCCCCCGGGG AGTCAGAGCA TCTGCTGGAA CCGGCAGAAG   540
   CTGAAAGGTC TCAGAGGAGG AGGCTGCTGG TTCCAGCAAA TGAAGGTGAT CCCACTGAGA   600
   CTCTGAGACA GTGCTTCGAT GACTTTGCAG ACTTGGTGCC CTTTGACTCC TGGGAGCCGC   660
   TCATGAGGAA GTTGGGCCTC ATGGACAATG AGATAAAGGT GGCTAAAGCT GAGGCAGCGG   720
   GCCACAGGGA CACCTTGTAC ACGATGCTGA TAAAGTGGGT CAACAAAACC GGGCGAGATG   780
   CCTCTGTCCA CACCCTGCTG GATGCCTTGG AGACGCTGGG AGAGAGACTT GCCAAGCAGA   840
   AGATTGAGGA CCACTTGTTG AGCTCTGGAA AGTTCATGTA TCTAGAAGGT AATGCAGACT   900
   CTGCCATGTC CTAAGTGTGA TTCTCTTCAG GAAGTCAGAC CTTCCCTGGT TTACCTTTTT   960
   TCTGGAAAAA GCCCAACTGG ACTCCAGTCA GTAGGAAAGT GCCACAATTG TCACATGACC  1020
   GGTACTGGAA GAAACTCTCC CATCCAACAT CACCCAGTGG AT                     1062

### (2) INFORMATION FOR SEQ ID NO: 4:

(i) SEQUENCE CHARACTERISTICS:
   (A) LENGTH: 303 amino acids
   (B) TYPE: amino acid
   (C) STRANDEDNESS: single
   (D) TOPOLOGY: linear
(ii) MOLECULE TYPE: protein
(xi) SEQUENCE DESCRIPTION: SEQ ID NO: 4:
   Asp Leu Leu Phe Cys Leu Arg Cys Thr Arg Cys Asp Ser Gly Glu Val
    1               5                  10                  15
   Glu Leu Ser Pro Cys Thr Thr Thr Arg Asn Thr Val Cys Gln Cys Glu
               20                  25                  30
   Glu Gly Thr Phe Arg Glu Glu Asp Ser Pro Glu Met Cys Arg Lys Cys
           35                  40                  45
   Arg Thr Gly Cys Pro Arg Gly Met Val Lys Val Gly Asp Cys Thr Pro
       50                  55                  60
   Trp Ser Asp Ile Glu Cys Val His Lys Glu Ser Gly Ile Ile Ile Gly
   65                  70                  75                  80
   Val Thr Val Ala Ala Val Val Leu Ile Val Ala Val Phe Val Cys Lys
                   85                  90                  95
   Ser Leu Leu Trp Lys Lys Val Leu Pro Tyr Leu Lys Gly Ile Cys Ser
               100                 105                 110
   Gly Gly Gly Gly Asp Pro Glu Arg Val Asp Arg Ser Ser Gln Arg Pro
           115                 120                 125
   Gly Ala Glu Asp Asn Val Leu Asn Glu Ile Val Ser Ile Leu Gln Pro
       130                 135                 140
   Thr Gln Val Pro Glu Gln Glu Met Glu Val Gln Glu Pro Ala Glu Pro
   145                 150                 155                 160
   Thr Gly Val Asn Met Leu Ser Pro Gly Glu Ser Glu His Leu Leu Glu
                   165                 170                 175
   Pro Ala Glu Ala Glu Arg Ser Gln Arg Arg Arg Leu Leu Val Pro Ala
               180                 185                 190
   Asn Glu Gly Asp Pro Thr Glu Thr Leu Arg Gln Cys Phe Asp Asp Phe
           195                 200                 205
   Ala Asp Leu Val Pro Phe Asp Ser Trp Glu Pro Leu Met Arg Lys Leu
       210                 215                 220
   Gly Leu Met Asp Asn Glu Ile Lys Val Ala Lys Ala Glu Ala Ala Gly
   225                 230                 235                 240
   His Arg Asp Thr Leu Tyr Thr Met Leu Ile Lys Trp Val Asn Lys Thr
                   245                 250                 255
   Gly Arg Asp Ala Ser Val His Thr Leu Leu Asp Ala Leu Glu Thr Leu
               260                 265                 270
   Gly Glu Arg Leu Ala Lys Gln Lys Ile Glu Asp His Leu Leu Ser Ser
               275                 280                 285
   Gly Lys Phe Met Tyr Leu Glu Gly Asn Ala Asp Ser Ala Met Ser
       290                 295                 300

### (2) INFORMATION FOR SEQ ID NO:5:

(i) SEQUENCE CHARACTERISTICS:
   (A) LENGTH: 1769 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:5:
   CCTCACTGAC TATAAAAGAA TAGAGAAGGA AGGGCTTCAG TGACCGGCTG CCTGGCTGAC     60
   TTACAGCAGT CAGACTCTGA CAGGATCATG GCTATGATGG AGGTCCAGGG GGGACCCAGC    120
   CTGGGACAGA CCTGCGTGCT GATCGTGATC TTCACAGTGC TCCTGCAGTC TCTCTGTGTG    180
   GCTGTAACTT ACGTGTACTT TACCAACGAG CTGAAGCAGA TGCAGGACAA GTACTCCAAA    240
   AGTGGCATTG CTTGTTTCTT AAAAGAAGAT GACAGTTATT GGGACCCCAA TGACGAAGAG    300
   AGTATGAACA GCCCCTGCTG GCAAGTCAAG TGGCAACTCC GTCAGCTCGT TAGAAAGATG    360
   ATTTTGAGAA CCTCTGAGGA AACCATTTCT ACAGTTCAAG AAAAGCAACA AAATATTTCT    420
   CCCCTAGTGA GAGAAAGAGG TCCTCAGAGA GTAGCAGCTC ACATAACTGG GACCAGAGGA    480
   AGAAGCAACA CATTGTCTTC TCCAAACTCC AAGAATGAAA AGGCTCTGGG CCGCAAAATA    540
   AACTCCTGGG AATCATCAAG GAGTGGGCAT TCATTCCTGA GCAACTTGCA CTTGAGGAAT    600
   GGTGAACTGG TCATCCATGA AAAAGGGTTT TACTACATCT ATTCCCAAAC ATACTTTCGA    660
   TTTCAGGAGG AAATAAAAGA AAACACAAAG AACGACAAAC AAATGGTCCA ATATATTTAC    720
   AAATACACAA GTTATCCTGA CCCTATATTG TTGATGAAAA GTGCTAGAAA TAGTTGTTGG    780
   TCTAAAGATG CAGAATATGG ACTCTATTCC ATCTATCAAG GGGGAATATT TGAGCTTAAG    840
   GAAAATGACA GAATTTTTGT TTCTGTAACA AATGAGCACT TGATAGACAT GGACCATGAA    900
   GCCAGTTTTT TCGGGGCCTT TTTAGTTGGC TAACTGACCT GGAAAGAAAA AGCAATAACC    960
   TCAAAGTGAC TATTCAGTTT TCAGGATGAT ACACTATGAA GATGTTTCAA AAAATCTGAC   1020
   CAAAACAAAC AAACAGAAAA CAGAAAACAA AAAAACCTCT ATGCAATCTG AGTAGAGCAG   1080
   CCACAACCAA AAAATTCTAC AACACACACT GTTCTGAAAG TGACTCACTT ATCCCAAGAA   1140
   AATGAAATTG CTGAAAGATC TTTCAGGACT CTACCTCATA TCAGTTTGCT AGCAGAAATC   1200
   TAGAAGACTG TCAGCTTCCA AACATTAATG CAATGGTTAA CATCTTCTGT CTTTATAATC   1260
   TACTCCTTGT AAAGACTGTA GAAGAAAGCG CAACAATCCA TCTCTCAAGT AGTGTATCAC   1320
   AGTAGTAGCC TCCAGGTTTC CTTAAGGGAC AACATCCTTA AGTCAAAAGA GAGAAGAGGC   1380
   ACCACTAAAA GATCGCAGTT TGCCTGGTGC AGTGGCTCAC ACCTGTAATC CCAACATTTT   1440
   GGGAACCCAA GGTGGGTAGA TCACGAGATC AAGAGATCAA GACCATAGTG ACCAACATAG   1500
   TGAAACCCCA TCTCTACTGA AAGTGCAAAA ATTAGCTGGG TGTGTTGGCA CATGCCTGTA   1560
   GTCCCAGCTA CTTGAGAGGC TGAGGCAGGA GAATCGTTTG AACCCGGGAG GCAGAGGTTG   1620
   CAGTGTGGTG AGATCATGCC ACTACACTCC AGCCTGGCGA CAGAGCGAGA CTTGGTTTCA   1680
   AAAAAAAAAA AAAAAAAAAA CTTCAGTAAG TACGTGTTAT TTTTTTCAAT AAAATTCTAT   1740
   TACAGTATGT CAAAAAAAAA AAAAAAAAA                                     1769

### (2) INFORMATION FOR SEQ ID NO:6:

(i) SEQUENCE CHARACTERISTICS:
   (A) LENGTH: 281 amino acids
   (B) TYPE: amino acid
   (C) STRANDEDNESS: single
   (D) TOPOLOGY: linear
(ii) MOLECULE TYPE: protein
(xi) SEQUENCE DESCRIPTION: SEQ ID NO:6:

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to Tumor Necrosis Factor Related family, hereinafter referred to as TR6.

### BACKGROUND OF THE INVENTION

Many biological actions, for instance, response to certain stimuli and natural biological processes, are controlled by factors, such as cytokines. Many cytokines act through receptors by engaging the receptor and producing an intracellular response.

For example, tumor necrosis factors (TNF) alpha and beta are cytokines which act through TNF receptors to regulate numerous biological processes, including protection against infection and induction of shock and inflammatory disease. The TNF molecules belong to the "TNF-ligand" superfamily, and act together with their receptors or counter-ligands, the "TNF-receptor" superfamily. So far, nine members of the TNF ligand superfamily have been identified and ten members of the TNF-receptor superfamily have been characterized.

Among the ligands there are included TNF-α, lymphotoxin-α (LT-α, also known as TNF-β), LT-β (found in complex heterotrimer LT-α2-β), FasL, CD40L, CD27L, CD30L, 4-1BBL, OX40L and nerve growth factor (NGF)). The superfamily of TNF receptors includes the p55TNF receptor, p75TNF receptor, TNF receptor-related protein, FAS antigen or APO-1, CD40, CD27, CD30, 4-1BB, OX40, low affinity p75 and NGF-receptor (Meager, A., Biologicals, 22:291-295 (1994)).

Many members of the TNF-ligand superfamily are expressed by activated T-cells, implying that they are necessary for T-cell interactions with other cell types which underlie cell ontogeny and functions. (Meager, A., supra).

Considerable insight into the essential functions of several members of the TNF receptor family has been gained from the identification and creation of mutants that abolish the expression of these proteins. For example, naturally occurring mutations in the FAS antigen and its ligand cause lymphoproliferative disease (Watanabe-Fukunaga, R., et al., Nature 356:314 (1992)), perhaps reflecting a failure of programmed cell death. Mutations of the CD40 ligand cause an X-linked immunodeficiency state characterized by high levels of immunoglubulin M and low levels of immunoglobulin G in plasma, indicating faulty T-cell-dependent B-cell activation (Allen, R.C. et al., Science 259:990 (1993)). Targeted mutations of the low affinity nerve growth factor receptor cause a disorder characterized by faulty sensory innovation of peripheral structures (Lee, K.F. et al, Cell 69:737 (1992)).

TNF and LT-α are capable of binding to two TNF receptors (the 55- and 75-kd TNF receptors). A large number of biological effects elicited by TNF and LT-α, acting through their receptors, include hemorrhagic necrosis of transplanted tumors, cytotoxicity, a role in endotoxic shock, inflammation, immunoregulation, proliferation and anti-viral responses, as well as protection against the deleterious effects of ionizing radiation. TNF and LT-α are involved in the pathogenesis of a wide range of diseases, including endotoxic shock, cerebral malaria, tumors, autoimmuine disease, AIDS and graft-host rejection (Beutler, B. and Von Huffel, C., Science 264:667-668 (1994)). Mutations in the p55 Receptor cause increased susceptibility to microbial infection.

Moreover, an about 80 amino acid domain near the C-terminus of TNFR1 (P55) and Fas was reported as the "death domain," which is responsible for transducing signals for programmed cell death (Tartaglia et al., Cell 74:845 (1993)).

The effects of TNF family ligands and TNF family receptors are varied and influence numerous functions, both normal and abnormal, in the biological processes of the mammalian system. There is a clear need, therefore, for identification and characterization of such receptors and ligands that influence biological activity, both normally and in disease states. In particular, there is a need to isolate and characterize novel members of the TNF receptor family.

This indicates that these receptors have an established, proven history as therapeutic targets. Clearly there is a need for identification and characterization of further receptors which can play a role in preventing, ameliorating or correcting dysfunctions or diseases, including, but not limited to, chronic and acute inflammation, arthritis, septicemia, autoimmune diseases (e.g. inflammatory bowel disease, psoriasis), transplant rejection, graft vs. host disease, infection, stroke, ischemia, acute respiratory disease syndrome, restenosis, brain injury, AIDS, Bone diseases, cancer (e.g. lymphoproliferative disorders), atheroschlerosis, and Alzheimers disease.

### SUMMARY OF THE INVENTION

In one aspect, the invention relates to TR6 polypeptides and recombinant materials and methods for their production.

In still another aspect, the invention relates to methods to identify antagonists using the materials provided by the invention

### DESCRIPTION OF THE INVENTION

### Definitions

The following definitions are provided to facilitate understanding of certain terms used frequently herein.

"TR6" refers, among others, to a polypeptide comprising the amino acid sequence set forth in SEQ ID NO:2, or an allelic variant thereof.

"Receptor Activity" or "Biological Activity of the Receptor" refers to the metabolic or physiologic function of said TR6 including similar activities or improved activities or these activities with decreased undesirable side-effects. Also included are antigenic and immunogenic activities of said TR6.

"TR6 gene" refers to a polynucleotide comprising the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., "Analysis for protein modifications and nonprotein cofactors", *Meth Enzymol* (1990) 182:626-646 and Rattan *et al*., "Protein Synthesis: Posttranslational Modifications and Aging", *Ann NY Acad Sci* (1992) 663 :48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to TR6 polypeptides. The TR6 polypeptides include the polypeptides of SEQ ID NOS:2 and 4; as well as polypeptides comprising the amino acid sequence of SEQ ID NO:2.

The TR6 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10,1-5, or 1-2 amino acids are substituted, deleted, or added in any combination.

The TR6 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to TR6 polynucleotides. TR6 polynucleotides include isolated polynucleotides which encode the TR6 polypeptides and fragments, and polynucleotides closely related thereto. More specifically, TR6 polynucleotide of the invention include a polynucleotide comprising the nucleotide sequence set forth in SEQ ID NO: 1 encoding a TR6 polypeptide of SEQ ID NO: 2, and polynucleotides having the particular sequences of SEQ ID NOS: and 3. TR6 polynucleotides further include a polynucleotide comprising a nucleotide sequence that has at least 80% identity to a nucleotide sequence encoding the TR6 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under TR6 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such TR6 polynucleotides.

TR6 of the invention is structurally related to other proteins of the Tumor Necrosis Factor Related family, as shown by the results of sequencing the cDNA encoding human TR6. The cDNA sequence of SEQ ID NO:1 contains an open reading frame (nucleotide numbers 94 to 1329) encoding a polypeptide of 411 amino acids of SEQ ID NO:2. The amino acid sequence of Table 1 (SEQ ID NO:2) has about 58% identity (using GAP (From GCG )) in 411 amino acid residues with DR4, the receptor for the ligand TRAIL. (Pan,G., O'Rourke,K., Chinnaiyan,A.M., Gentz,R., Ebner,R., Ni,J. and Dixit,V.M., Science 276, 111-113 (1997)). The nucleotide sequence of Table I (SEQ ID NO:1) has about 70% identity (using GAP (from GCG)) in 1335 nucleotide residues with DR4, the receptor for the ligand TRAIL. TR6 contains a death domain (amino acids 290 to 324 in SEQ ID NO:2) which is 64% identical to the death domain of the human Death receptor 4 (DR4) (Pan,G., O'Rourke,K., Chinnaiyan,A.M., Gentz,R., Ebner,R., Ni,J. and Dixit,V.M., Science 276, 111-113 (1997)), 35.7% identical to the death domain of the human Death receptor 3 (DR3) (A.M. Chinnaiyan, et al, Science 274 (5289), 990-992 (1996)), 32.7% identical to the death domain of human TNFR-1, and 19.6% identical to the death domain of CD95 (Fas) (I. Cascino, J. Immunol. 154 (6), 2706-2713 (1995)).

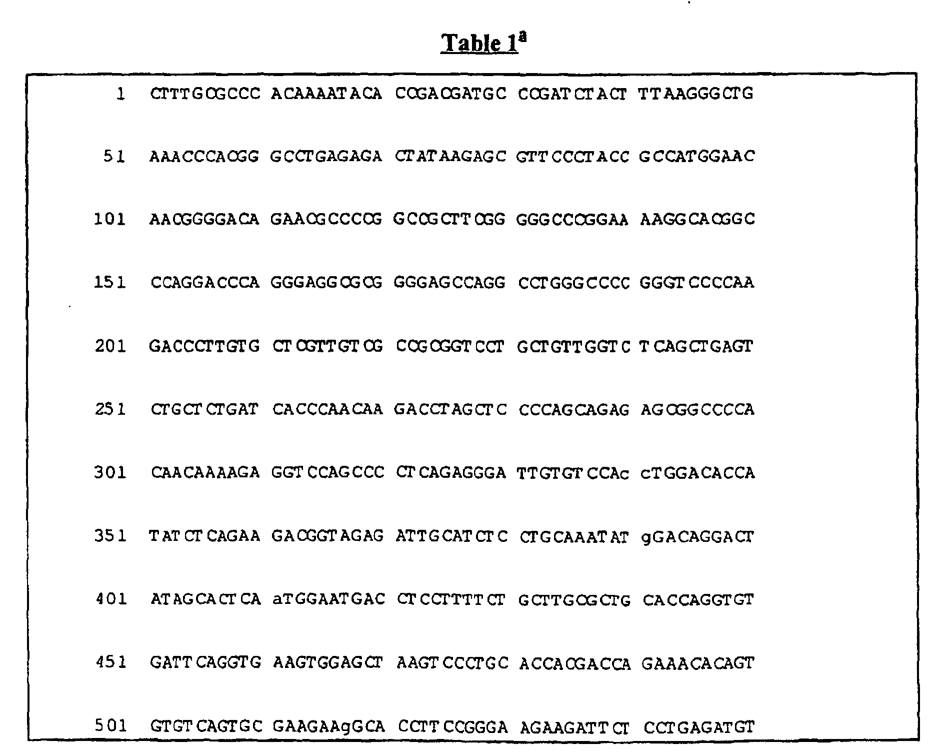

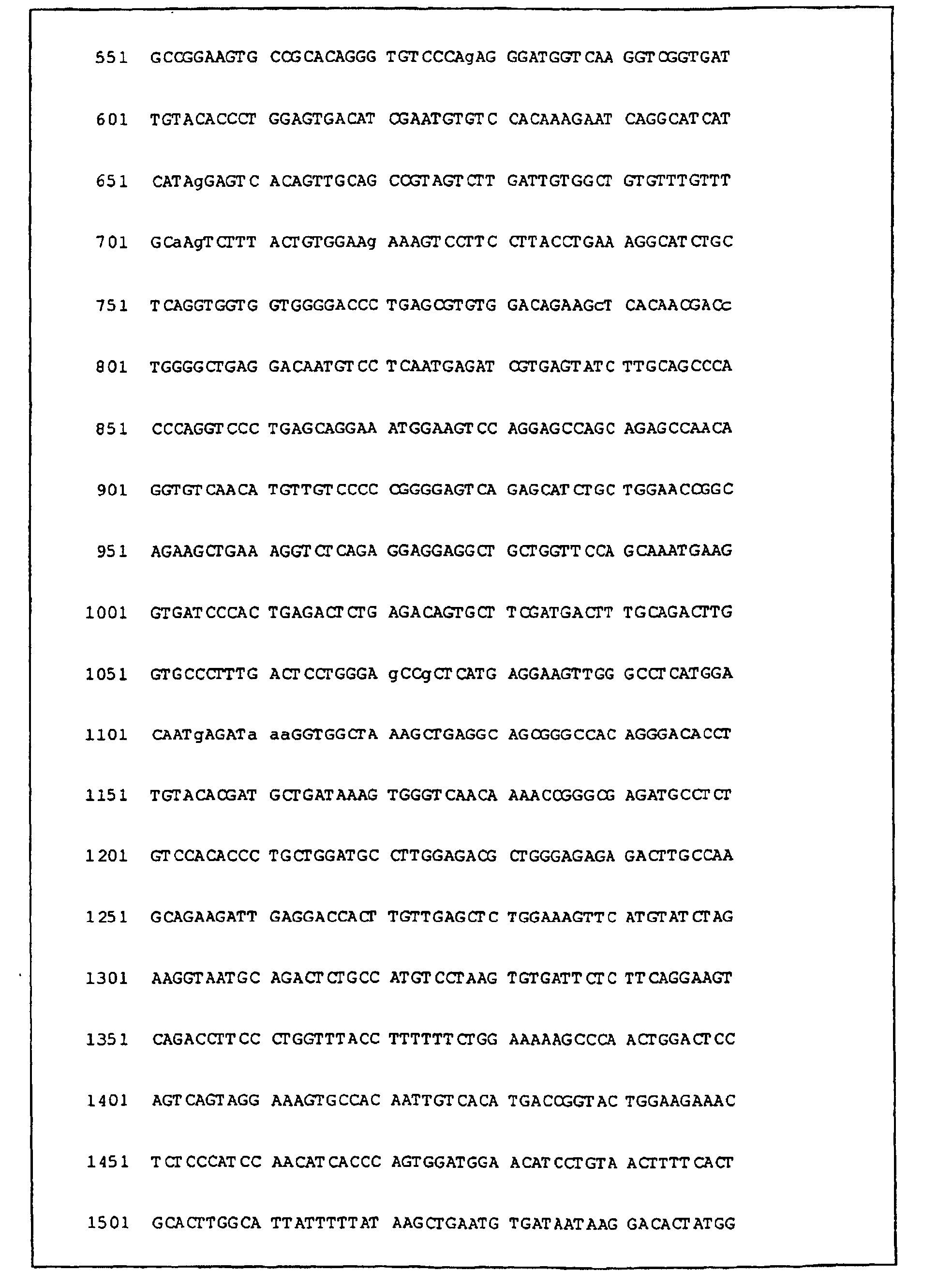

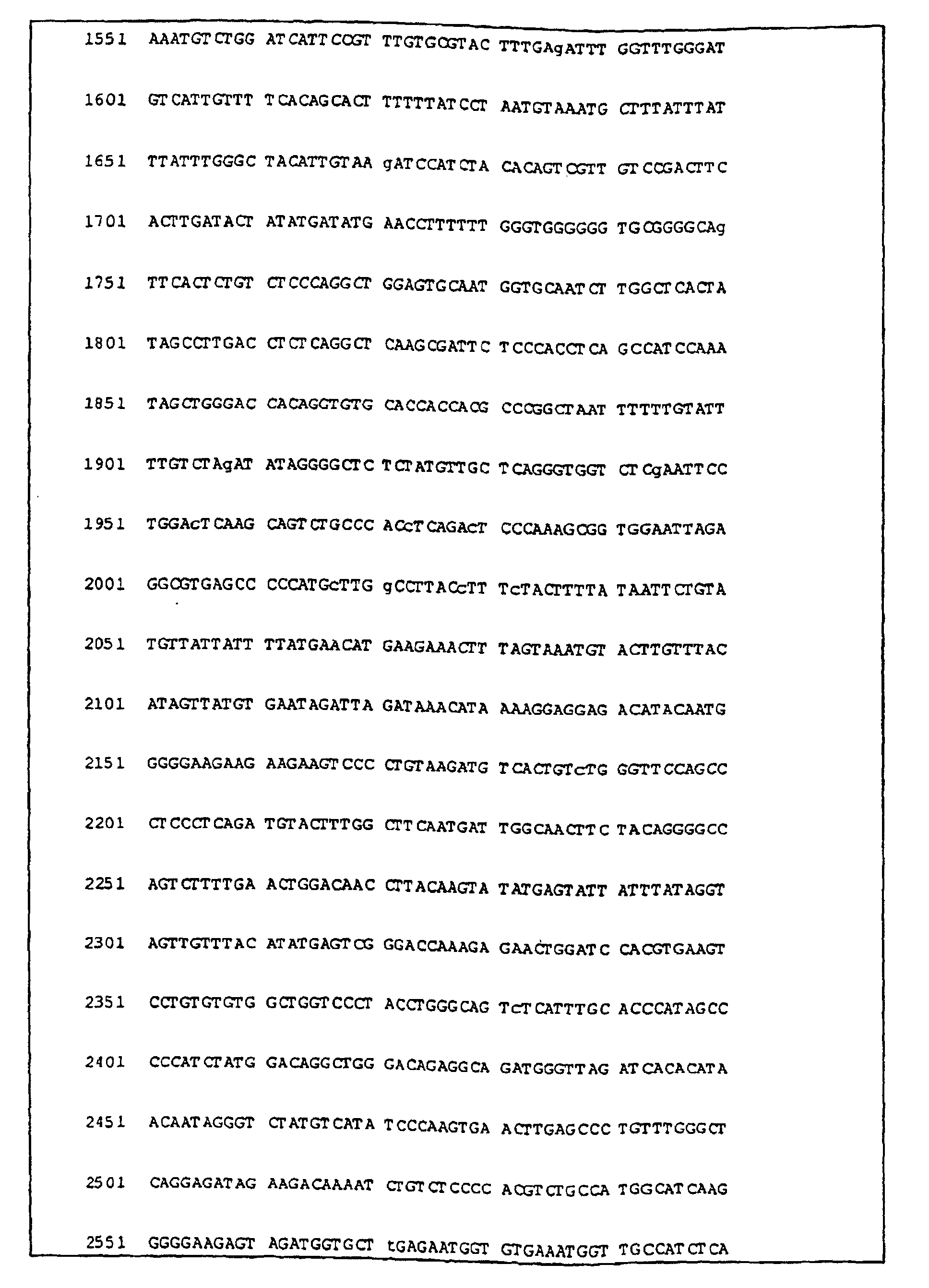

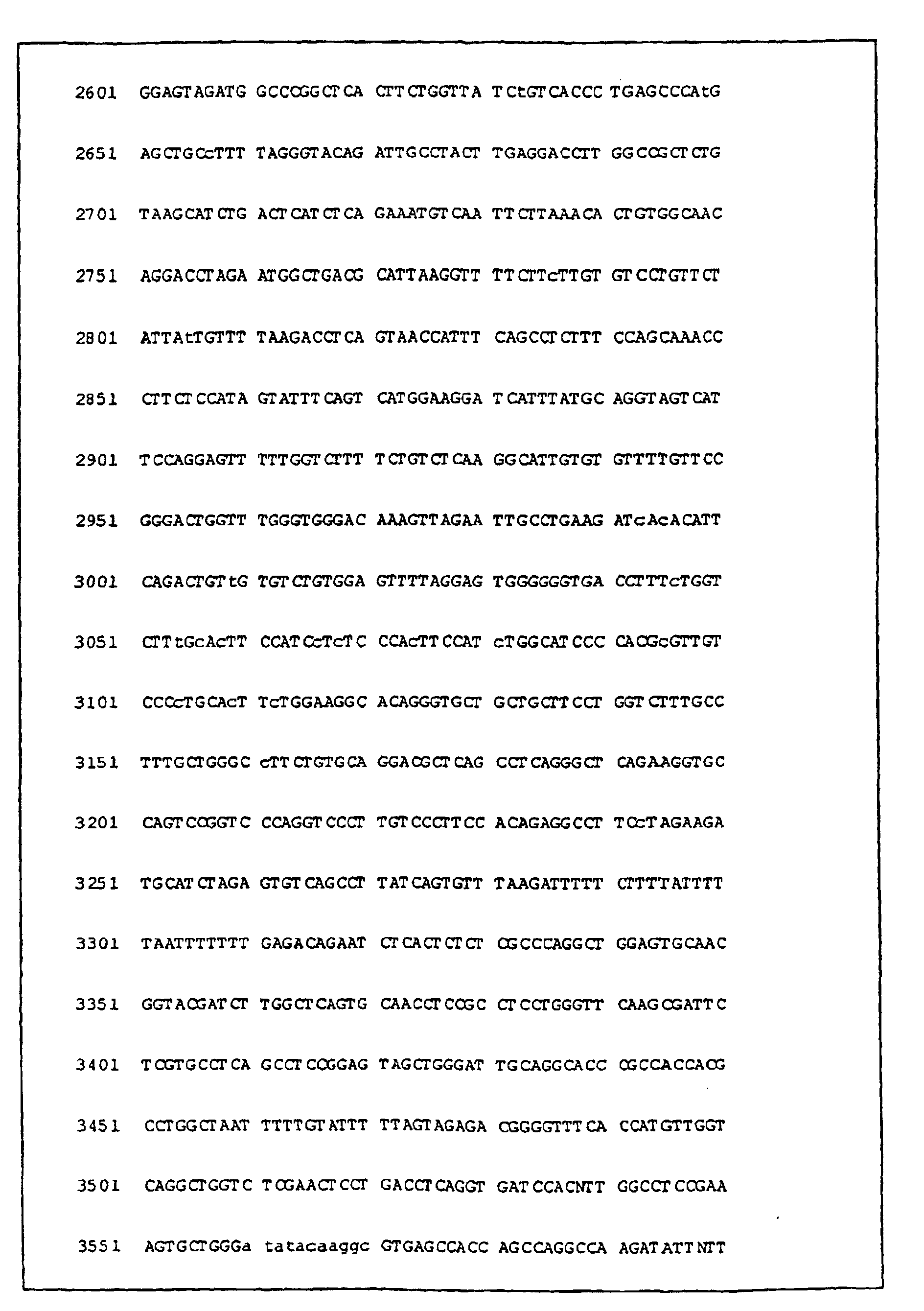

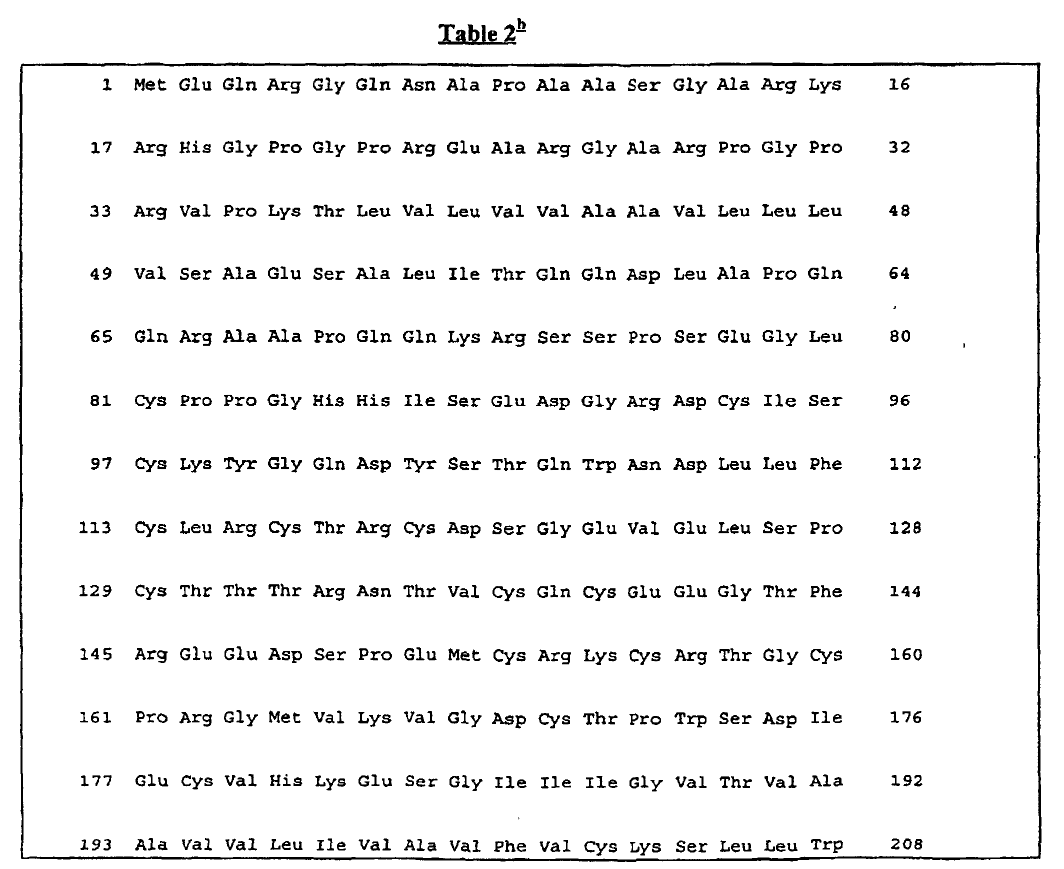

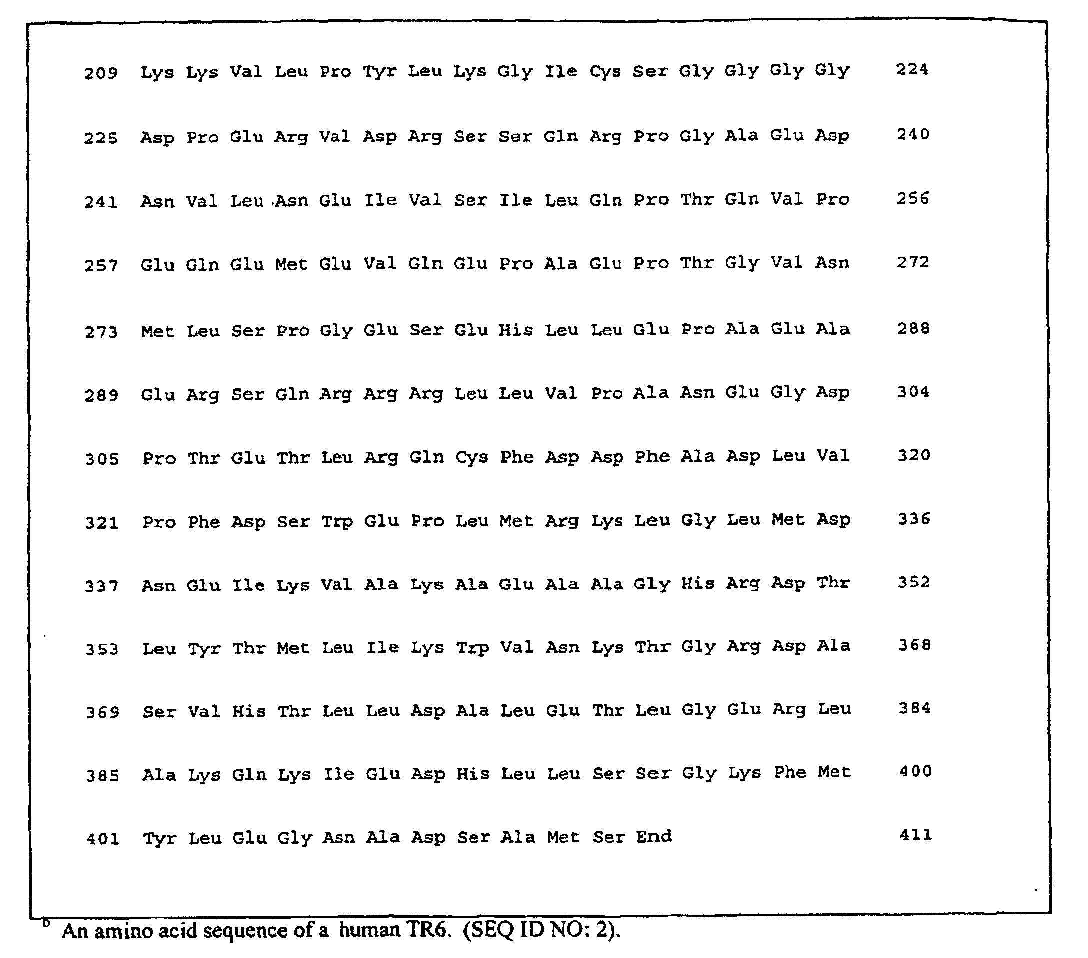

One polynucleotide of the present invention encoding TR6 may be obtained using standard cloning and screening, from a cDNA library derived from mRNA in cells of human of human thymus stromal cells, monocytes, peripheral blood lymphocytes, primary dendritic, and bone marrow cells using the expressed sequence tag (EST) analysis (Adams, M.D., *et al*. *Science* (1991) 252:1651-1656; Adams, M.D. *et al*., *Nature,* (1992) *355*:632-634; Adams, M.D., *et al*., *Nature* (1995) 377 Supp:3-174). Polynucleotides ofthe invention can also be obtained from natural sources such as genomic DNA libraries or can be synthesized using well known and commercially available techniques.

The nucleotide sequence encoding TR6 polypeptide of SEQ ID NO:2 may be identical to the polypeptide encoding sequence contained in Table 1 (nucleotide number 94 to 1329 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of TR6 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., *Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding TR6 variants comprising the amino acid sequence of TR6 polypeptide of Table 1 (SEQ ID NO:2) in which several, 5-10, 1-5, 1-3, 1-2 or 1 amino acid residues are substituted, deleted or added, in any combination. Among the preferred polynucleotides of the present invention is contained in Table 3 (SEQ ID NO: 3) encoding the amino acid sequence of Table 4 (SEQ ID NO: 4).

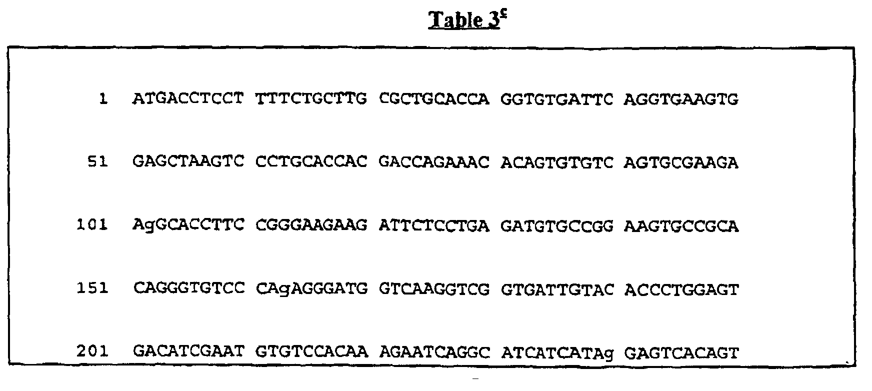

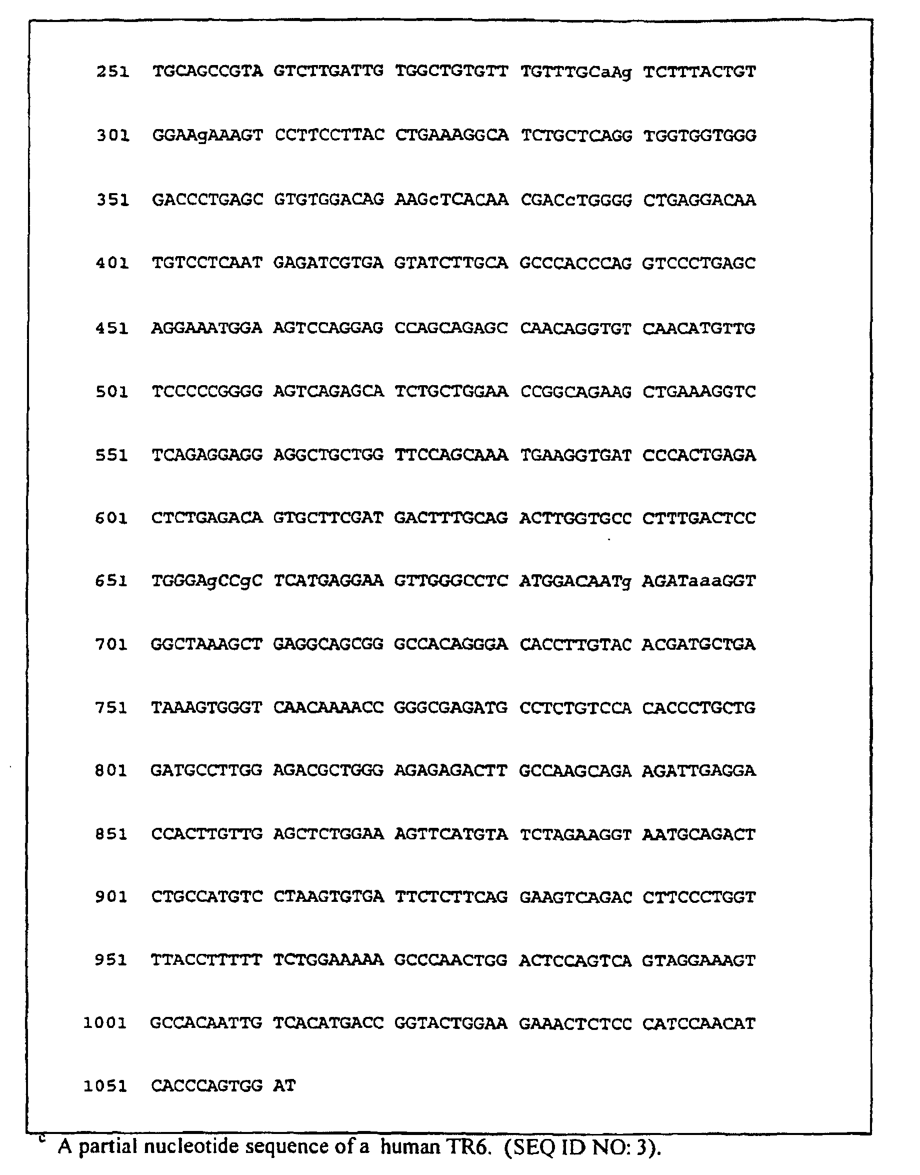

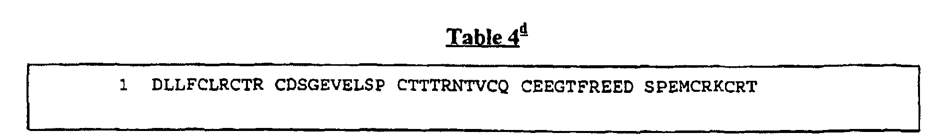

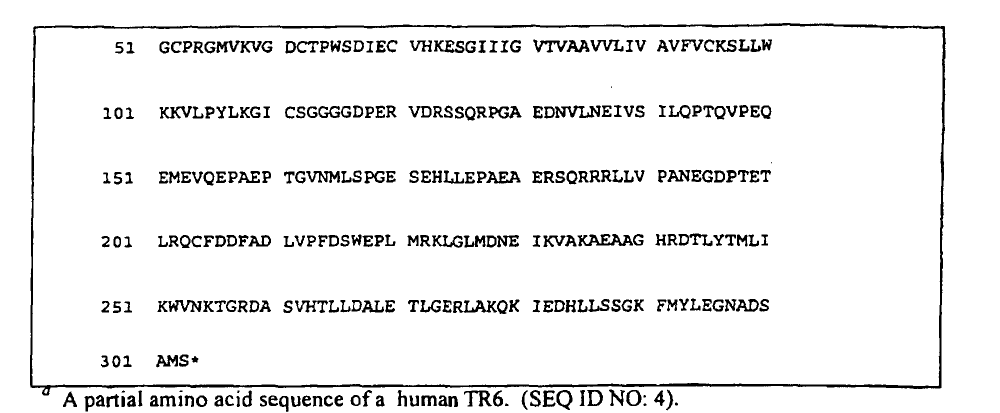

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, including that of SEQ ID NO:3, may be used as hybridization probes for cDNA and genomic DNA, to isolate full-length cDNAs and genomic clones encoding TR6 and to isolate cDNA and genomic clones of other genes that have a high sequence similarity to the TR6 gene. Such hybridization techniques are known to those of skill in the art. Typically these nucleotide sequences are 80% identical, preferably 90% identical, more preferably 95% identical to that of the referent.

In one embodiment, to obtain a polynucleotide encoding TR6 polypeptide comprises the steps of screening an appropriate library under stringent hybridization conditions with a labeled probe having the SEQ ID NO: 1 or a fragment thereof, including that of SEQ ID NO: 3, and isolating full-length cDNA and genomic clones containing said polynucleotide sequence. Such hybridization techniques are well known to those of skill in the art. Thus in another aspect, TR6 polynucleotides of the present invention further include a nucleotide sequence comprising a nucleotide sequence that hybridize under stringent condition to a nucleotide sequence having SEQ ID NO: 1 or a fragment thereof, including that of SEQ ID NO:3. Also included with TR6 polypeptides are polypeptide comprising amino acid sequence encoded by nucleotide sequence obtained by the above hybridization condition. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washine the filters in 0.1x SSC at about 65°C.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et aL, *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells *and Aspergillus* cells; insect cells such as *Drosophila* S2 and *Spodoptera* Sf9 cells; animal cells such as CHO, COS, HeLa, C 127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al*., *MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the TR6 polypeptide is to be expressed for use in screening assays, generally, it is preferred that the polypeptide be produced at the surface of the cell. In this event, the cells may be harvested prior to use in the screening assay. If TR6 polypeptide is secreted into the medium, the medium can be recovered in order to recover and purify the polypeptide; if produced intracellularly, the cells must first be lysed before the polypeptide is recovered.

TR6 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Screening Assays

We have now discovered that TL2 of SEQ ID NO: 5 (otherwise known as TRAIL, Immunity (6):673-682 (1995)) is a ligand of TR6. Thus, the TR6 polypeptide of the present invention, and one of its ligands, TL2 may be employed in a screening process for compounds which bind the receptor, or its ligand, and which inhibit activation of (antagonists) the receptor polypeptide of the present invention, or its ligand TL2. Thus, polypeptides of the invention may be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al., Current Protocols in Immunology* 1(2):Chapter 5 (1991).

TR6 polypeptides are responsible for many biological functions, including many pathologies. Accordingly, it is desirable to find compounds and drugs which stimulate TR6 on the one hand and which can inhibit the function of TR6 or remove TR6 expressing cells on the other hand. Antagonists, or agents which remove TR6 expressing cells, may be employed for a variety of therapeutic and prophylactic purposes for such conditions as chronic and acute inflammation, arthritis, septicemia, autoimmune diseases (e.g. inflammatory bowel disease, psoriasis), transplant rejection, graft vs. host disease, infection, stroke, ischemia, acute respiratory disease syndrome, restenosis, brain injury, AIDS, Bone diseases, cancer (e.g. lymphoproliferative disorders), atheroschlerosis, and Alzheimers disease.

Candidate compounds may be identified using assays to detect compounds which inhibit binding of TL2 to TR6 in either cell-free or cell based assays. Suitable cell-free assays may be readily determined by one of skill in the art. For example, an ELISA format may be used in which purified TR6, or a purified derivative of TR6, containing the extracellular domain of TR6, is immobilized on a suitable surface, either directly or indirectly (e.g., via an antibody to TR6) and candidate compounds are identified by their ability to block binding of purified TL2 to TR6. The binding of TL2 to TR6 could be detected by using a label directly or indirectly associated with TL2. Suitable detection systems include the streptavidin horseradish peroxidase conjugate, or direct conjugation by a tag, e.g., fluorescein. Conversely, purified TL2 may be immobilized on a suitable surface, and candidate compounds identified by their ability to block binding of purified TR6 to TL2. The binding of TR6 to TL2 could be detected by using a label directly or indirectly associated with TR6. Many other assay formats are possible that use the TR6 protein and its ligands.

Suitable cell based assays may be readily determined by one of skill in the art. In general, such screening procedures involve producing appropriate cells which express the receptor polypeptide of the present invention on the surface thereof. Such cells include cells from mammals, yeast, *Drosophila* or *E. coli.* Cells expressing the receptor (or cell membrane containing the expressed receptor) are then contacted with a known ligand, such as TL2, or test compound to observe binding, or stimulation or inhibition of a functional response. The assays may simply test binding of a candidate compound wherein adherence to the cells bearing the receptor is detected by means of a label directly or indirectly associated with the candidate compound or in an assay involving competition with a labeled competitor, such as the ligand TL2. Further, these assays may test whether the candidate compound results in a signal generated by activation of the receptor or its ligand (e.g. TL2)using detection systems appropriate to the cells bearing the receptor or its ligand and fusion proteins thereof at their surfaces. Typical fusion partners include fusing the extracellular domain of the receptor or ligand with the intracellular tyrosine kinase domain of a second receptor. Inhibitors of activation are generally assayed in the presence of a known agonist, such as the ligand TL2, and the effect on activation by the agonist by the presence of the candidate compound is observed. Standard methods for conducting such screening assays are well understood in the art.

Examples of potential TR6 antagonists include antibodies or, in some cases, oligonucleotides or proteins which are closely related to the ligand of the TR6, e.g., a fragment of the ligand TL2, or small molecules which bind to the receptor, or its ligand, but do not elicit a response, so that the activity of the receptor is prevented.

The nucleotide sequence of TL2 (SEQ ID NO:5) (published by Immunex Research and Development Corporation, Seattle, Washington as TNF-related apoptosis-inducing ligand (TRAIL) TWiley SR, et al. Immunity (6):673-682 (1995)) is as follows.

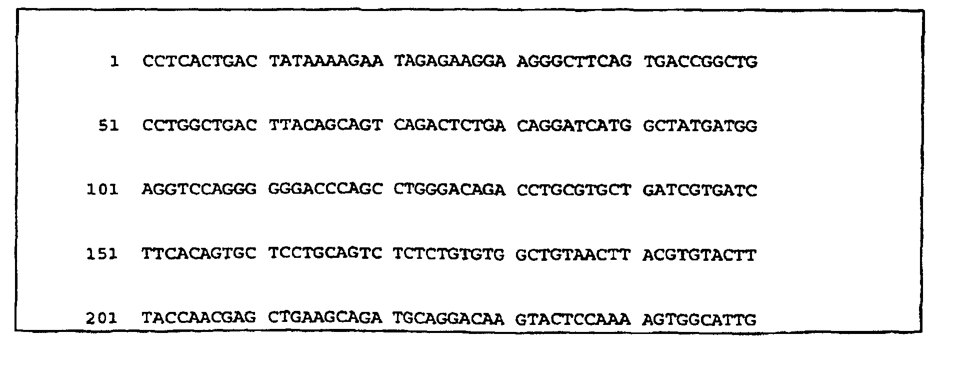

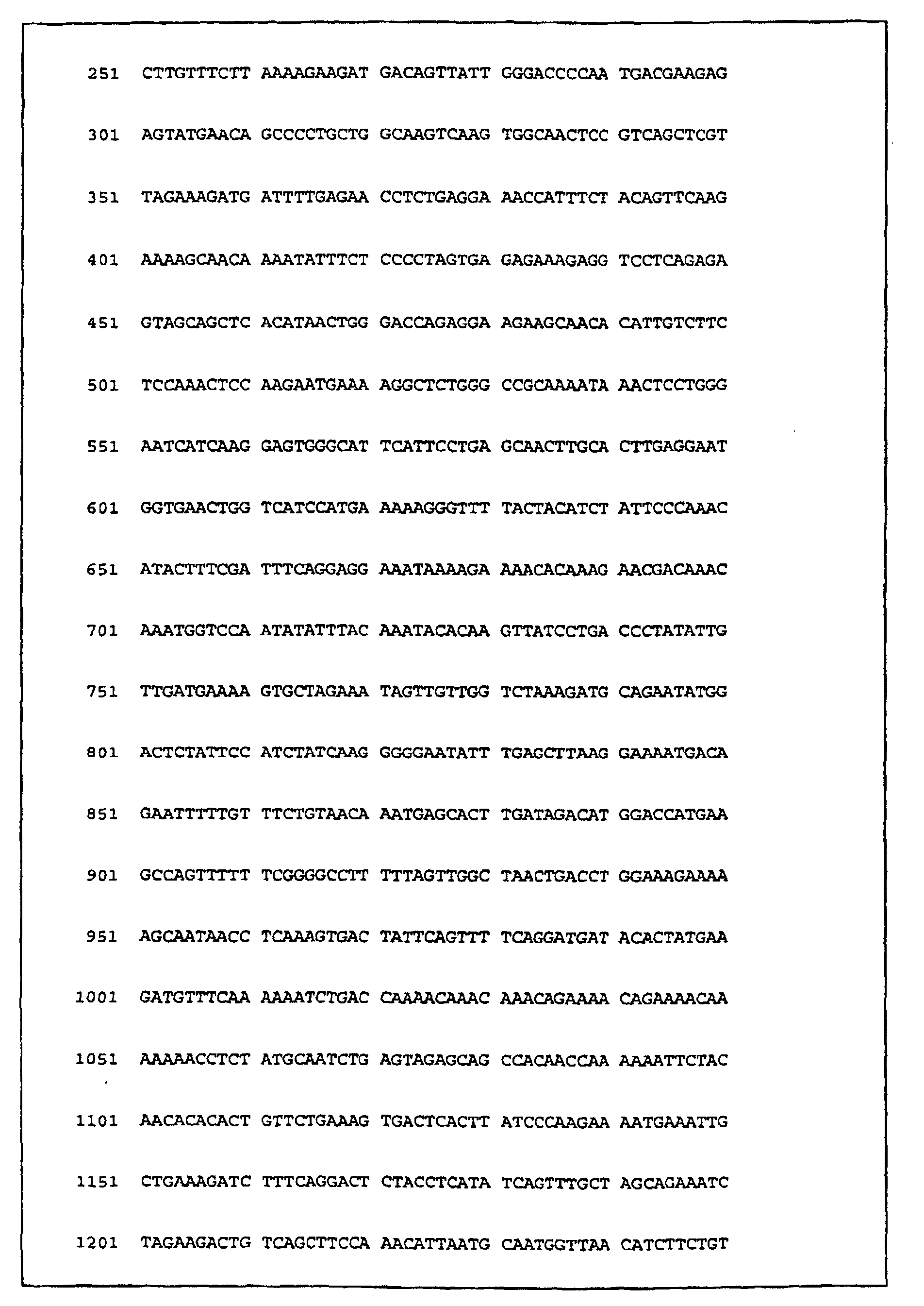

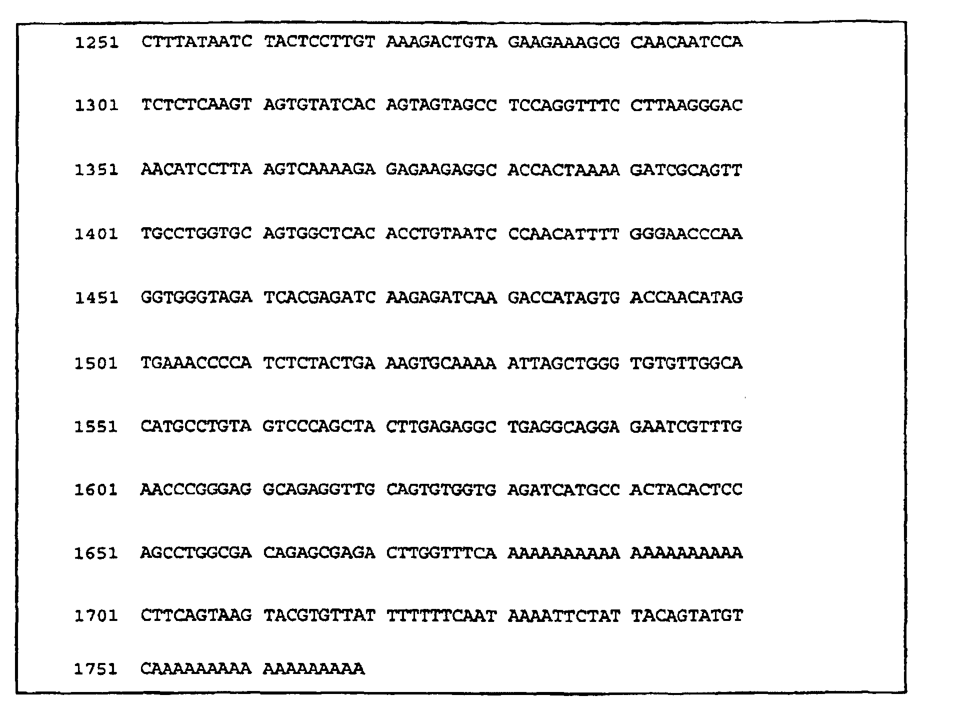

The amino acid sequence of TL2 (SEQ ID NO:6) (published by Immunex Research and Development Corporation, Seattle, Washington as TNF-related apoptosis-inducing ligand (TRAIL) TWiley SR, et al. Immunity (6):673-682 (1995)) is as follows:

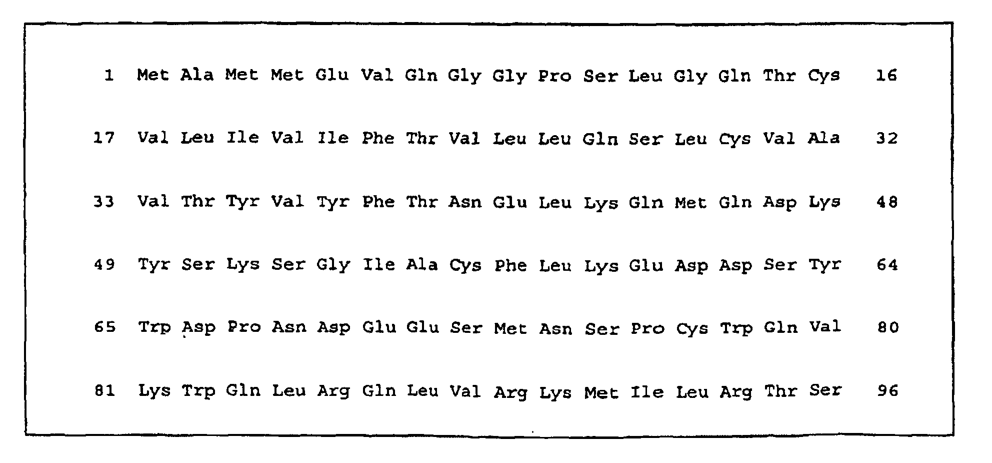

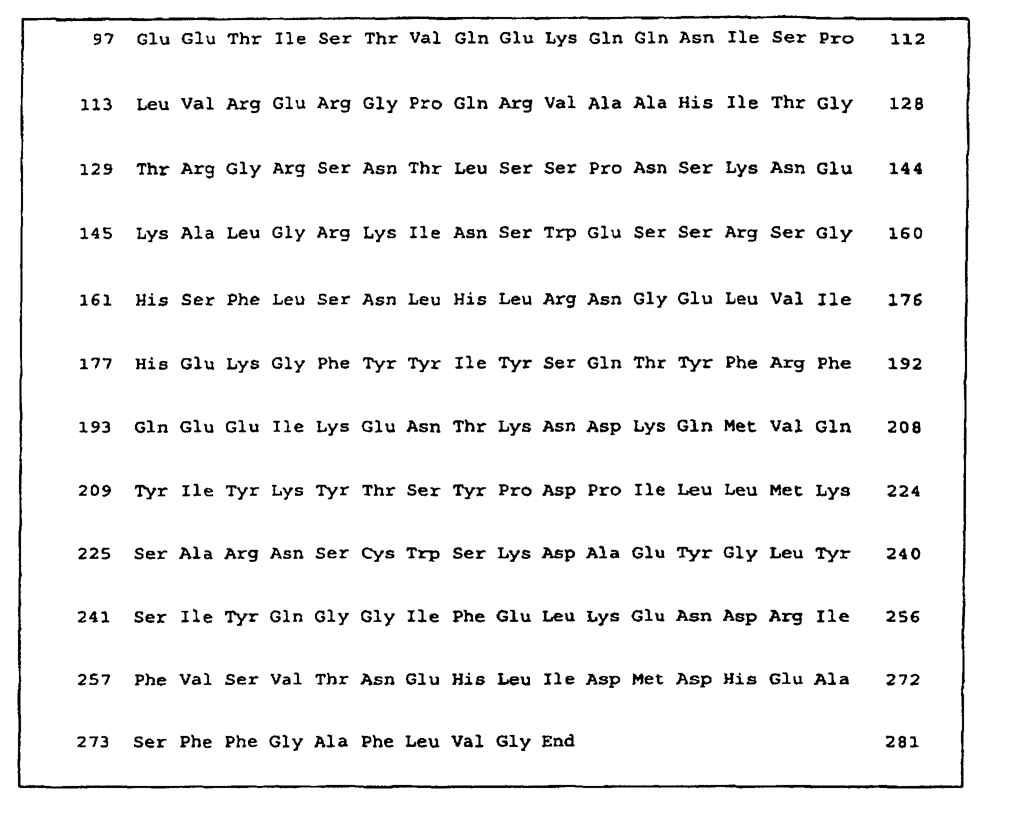

### Examples

The examples below are carried out using standard techniques, which are well known and routine to those of skill in the art, except where otherwise described in detail. The examples illustrate, but do not limit the invention.

### Example 1

Two ESTs (EST#1760054 and EST#1635744) with sequence similarity to the human TNF receptor were discovered in a commercial EST database. Analysis of the two nucleotide sequences (3,466 bp and 2,641 bp respectively), revealed each was a partial sequence of the complete cDNA sequence, overlapping, with 100% identity, 2,226 bp at the nucleotide level. Together, the two sequences encompassed the complete predicted cDNA sequence of 3,881 bp, and encoded an open reading frame for a novel member of the TNF receptor superfamily and named TR6. The predicted protein is 411 amino acids long with a hydrophobic membrane spanning region indicating that at least one form of TR6 is expressed as a membrane bound protein. Comparison of TR6 protein sequence, with other TNF receptor family proteins indicates that it has two of the cysteine-rich repeats characteristic of the extracellular domains of this family, and an intracellular death domain.

### Northern blot of TR6.

Various tissues and cell lines were screened for mRNA expression by Northern blot. RNA was prepared from cells and cell lines using Tri-Reagent (Molecular Research Center Inc., Cincinnati, OH), run in denaturing agarose gels (Sambrook et al., Molecular Cloning: a laboratory manual, 2nd Ed. Cold Spring Harbor Lab Press, NY (1989)) and transfered to Zeta-probe nylon membrane (Biorad, Hercules, CA.) via vacuum blotting in 25mM NaOh for 90 min. After neutralization for 5-10 minutes with 1M tris-HCl, pH 7.5 containing 3M NaCl, the blots were prehybridized with 50% formamide, 8% dextran sulfate, 6XSSPE, 0.1%SDS and 100mg/ml of sheared and dentured salmon sperm DNA for at least 30 min. At 42°C. cDNA probes were labeled with 32P-CTP by random priming (Statagene, La Jolla, CA), briefly denatured with 0.25M NaOH and added to the prehybridization solution. After a further incubation for at least 24h at 42°C, the blots were washed in high stringency conditions and exposed to X-ray film.

Very high expression of TR6 RNA was detected in aortic endothelial cells. High expression was also detected in monocytes. Low expression was detected in bone marrow and CD4+ activated PBLs. Very low, but detectable levels of TR6 RNA was expressed in CD19+ PBLs, CD8+ PBLs (both activated and unstimulated), and unstimulated CD4+ PBLs.

In hematopoietic cell lines, low levels of TR6 RNA was expressed in HL60 (promyelocyte), KG1a (promyeloblast) and KG1 (myeloblast) cell lines. Very low but detectable levels of TR6 RNA was expressed in U937 (monoblast) and THP-1 (monocyte) cell lines.

The major RNA form is 3.8 kb in size.

## Claims

1. An isolated polynucleotide comprising a nucleotide sequence that encodes the TR6 polypeptide of SEQ ID NO:2; or a nucleotide sequence complementary to said nucleotide sequence.

2. The polynucleotide of claim 1 which is DNA or RNA.

3. The polynucleotide of claim 1 wherein said nucleotide sequence is at least 80% identical to that contained in SEQ ID NO:1.

4. The polynucleotide of claim 3 wherein said nucleotide sequence comprises the TR6 polypeptide encoding sequence contained in SEQ ID NO:1.

5. The polynucleotide of claim 3 which is the polynucleotide of SEQ ID NO: 1.

6. A fragment of the polynucleotide claimed in any preceding claim, which fragment consists of the polynucleotide sequence set out in SEQ ID NO:3.

7. A DNA or RNA molecule comprising an expression system, wherein said expression system is capable of producing a TR6 polypeptide comprising the amino acid sequence of SEQ ID NO:2 when said expression system is present in a compatible host cell.

8. A host cell comprising the expression system of claim 7.

9. A process for producing a TR6 polypeptide comprising culturing a host of claim 8 under conditions sufficient for the production of said polypeptide and recovering the polypeptide from the culture.

10. A process for producing a cell which produces a TR6 polypeptide thereof comprising transforming or transfecting a host cell with the expression system of claim 7 such that the host cell, under appropriate culture conditions, produces a TR6 polypeptide.

11. A TR6 polypeptide comprising the amino acid sequence of SEQ ID NO:2.

12. A TR6 polypeptide consisting of the amino acid sequence of SEQ ID NO:2.

13. A fragment of a TR6 polypeptide consisting of the amino acid sequence of SEQ ID NO:4.

14. A method for identifying an antagonist to the TR6 polypeptide of claim 11 or claim 12, comprising the steps of:
(a) contacting a cell which produces a TR6 polypeptide with labelled TL2 ligand; and
(b) determining whether binding of said labelled TL2 ligand is diminished in the presence of a candidate compound.

## Patentansprüche

1. Isoliertes Polynucleotid, das eine Nucleotidsequenz, die das TR6-Polypeptid des Sequenzprotokolls SEQ ID NO:2 codiert, oder eine zu dieser Nucleotidsequenz komplementäre Nucleotidsequenz aufweist.

2. Polynucleotid nach Anspruch 1, das eine DNA oder eine RNA darstellt.

3. Polynucleotid nach Anspruch 1, wobei die Nucleotidsequenz zu mindestens 80 % mit der im Sequenzprotokoll SEQ ID NO:1 enthaltenen Nucleotidsequenz identisch ist.

4. Polynucleotid nach Anspruch 3, wobei die Nucleotidsequenz die im Sequenzprotokoll SEQ ID NO:1 enthaltene Sequenz enthält, die das TR6-Polypeptid codiert.

5. Polynucleotid nach Anspruch 3, welches das Polynucleotid des Sequenzprotokolls SEQ ID NO:1 ist.

6. Fragment des in einem der vorhergehenden Ansprüche beanspruchten Polynucleotids, wobei das Fragment aus der im Sequenzprotokoll SEQ ID NO:3 aufgeführten Sequenz besteht.

7. DNA- oder RNA-Molekül, das ein Expressionssystem aufweist, wobei das Expressionssystem zur Erzeugung eines TR6-Polypeptids befähigt ist, das die Aminosäuresequenz des Sequenzprotokolls SEQ ID NO:2 aufweist, wenn das Expressionssystem in einer kompatiblen Wirtszelle vorliegt.

8. Wirtszelle, die das Expressionssystem von Beispiel 7 aufweist.

9. Verfahren zur Erzeugung eines TR6-Polypeptids, das die Kultivierung von Wirtszellen nach Anspruch 8 unter Bedingungen, die für die Erzeugung dieses Polypeptids ausreichend sind, und die Gewinnung des Polypeptids aus der Kultur umfasst.

10. Verfahren zur Herstellung einer Zelle, die ein TR6-Polypeptid erzeugt, das die Transformation oder Transfektion einer Wirtszelle mit dem Expressionssystem nach Anspruch 7 in der Weise umfasst, dass die Wirtszelle, unter geeigneten Kulturbedingungen, ein TR6-Polypeptid erzeugt.

11. TR6-Polypeptid, das die Aminosäuresequenz des Sequenzprotokolls SEQ ID NO:2 aufweist.

12. TR6-Polypeptid, das aus der Aminosäuresequenz des Sequenzprotokolls SEQ ID NO:2 besteht.

13. Fragment eines TR6-Polypeptids, das aus der Aminosäuresequenz des Sequenzprotokolls SEQ ID NO:4 besteht.

14. Verfahren zur Identifizierung eines Antagonisten des TR6-Polypeptids nach Anspruch 11 oder 12, das folgende Schritte umfasst:
(a) Inkontaktbringen einer Zelle, die ein TR6-Polypeptid erzeugt, mit einem markierten TL2-Liganden und
(b) Ermittlung, ob die Bindung des markierten TL2-Liganden in Gegenwart einer Kandidatenverbindung verringert ist.

## Revendications

1. Polynucléotide isolé comprenant une séquence nucléotidique codant le polypeptide TR6 de SEQ ID NO:2 ou séquence nucléotidique complémentaire de ladite séquence nucléotidique.

2. Polynucléotide selon la revendication 1, qui est de l'ADN ou de l'ARN.

3. Polynucléotide selon la revendication 1, dans lequel ladite séquence nucléotidique est au moins 80 % identique à celle contenue dans SEQ ID NO:1.

4. Polynucléotide selon la revendication 3, dans lequel ladite séquence nucléotidique comprend le polypeptide TR6 codant la séquence contenue dans SEQ ID NO:1.

5. Polynucléotide selon la revendication 3, qui est le polynucléotide de SEQ ID NO:1.

6. Fragment du polynucléotide revendiqué dans l'une quelconque des revendications précédentes, ledit fragment consistant en la séquence polynucléotidique définie dans SEQ ID NO:3.

7. Molécule d'ADN ou d'ARN comprenant un système d'expression, dans laquelle ledit système d'expression est capable de produire un polypeptide TR6 comprenant la séquence d'acides aminés de SEQ ID NO:2 lorsque ledit système d'expression est présent dans une cellule hôte compatible.

8. Cellule hôte comprenant le système d'expression de la revendication 7.

9. Procédé de production d'un polypeptide TR6 comprenant la mise en culture d'un hôte de la revendication 8 dans des conditions suffisantes pour la production dudit polypeptide et la récupération du polypeptide à partir de la culture.

10. Procédé de production d'une cellule qui produit un polypeptide TR6 de celle-ci comprenant la transformation ou la transfection d'une cellule hôte avec le système d'expression de la revendication 7 de façon à ce que la cellule hôte, dans des conditions de culture appropriées, produise un polypeptide TR6.

11. Polypeptide TR6 comprenant la séquence d'acides aminés de SEQ ID NO:2.

12. Polypeptide TR6 consistant en la séquence d'acides aminés de SEQ ID NO:2.

13. Fragment d'un polypeptide TR6 consistant en la séquence d'acides aminés de SEQ ID NO:4.

14. Méthode d'identification d'un antagoniste du polypeptide TR6 de la revendication 11 ou 12, comprenant les étapes de :
(a) mettre en contact une cellule qui produit un polypeptide TR6 avec un ligand TL2 marqué ; et
(b) déterminer si la liaison dudit ligand TL2 marqué est diminuée en présence d'un composé candidat.
